# EUROPÄISCHE PATENTANMELDUNG

(11) **EP 2 992 859 A1**
(43) Veröffentlichungstag der Anmeldung: **09.03.2016**
(21) Anmeldenummer: 14183199.0
(22) Anmeldetag: 02.09.2014
(51) Int. Cl.: A61F 2/44

(54) **Kontinuierlich verstellbares Zwischenwirbelimplantat**

(71) Anmelder: FACET-LINK Inc., Rockaway NJ 07866 (US)
(72) Erfinder: Dmuschewsky, Klaus, 22397 Hamburg (DE)
(74) Vertreter: Glawe, Delfs, Moll

(57) **Zusammenfassung**

Die Erfindung betrifft ein Zwischenwirbelimplantat umfassend mindestens zwei gegenüberliegende voneinander weg weisende Anlageflächen (10, 10') zur Anlage an zueinander weisende Wirbelkörperflächen (30, 30') zweier benachbarter Wirbel (3, 3'), wobei ein Abstand der beiden Anlageflächen (10, 10') mittels einer Expansionseinrichtung veränderlich ist, die zwischen den Anlageflächen (10, 10') angeordnet ist und mindestens ein Betätigungselement (12) aufweist, wobei die Expansionseinrichtung mindestens zwei über eine Drehachse (13) scherenartig miteinander verbundene verschwenkbare Hebelelemente (14, 15) aufweist und die Expansionseinrichtung mindestens eine Spreizkomponente (16, 16'), die zur Aufspreizung der Hebelelemente (14, 15) ausgebildet ist, und mindestens ein Sicherungselement (17) umfasst, wobei das Sicherungselement (17) zur Fixierung der minimalen Aufspreizung der Hebelelemente (14, 15) ausgebildet ist. Der Aufbau des Zwischenwirbelimplantats (1) bewirkt eine hohe Belastbarkeit und eine hohe Kraftentwicklung sowie eine ausreichend große Gesamthöhenverstellung bei der Expansion. Weiter kann die Einstellung eines Lordosewinkels vorgenommen werden.

## Beschreibung

Die Erfindung betrifft ein Zwischenwirbelimplantat umfassend mindestens zwei gegenüberliegende voneinander wegweisende Anlageflächen zur Anlage an zueinander weisende Wirbelkörperflächen zweier benachbarter Wirbel und mindestens eine zwischen den Anlageflächen angeordnete Expansionseinrichtung wobei die Expansionseinrichtung mindestens ein Betätigungselement aufweist.

Verschleiß oder krankhafte Veränderungen an der Wirbelsäule können die Bandscheiben degenerieren. Soweit eine konservative Therapie mit Medikation und/oder Physiotherapie nicht greift, ist mitunter eine operative Behandlung indiziert. Dazu ist es bekannt, ein bewegliches oder unbewegliches Implantat in den Wirbelzwischenraum mit der degenerierten Bandscheibe einzuführen. Dieses übernimmt die Trag- und Stützfunktion der degenerierten Bandscheibe. Ein unbewegliches Implantat wird auch als "Fusionsimplantat" bezeichnet, da es eine Relativbewegung der benachbarten Wirbel verhindert.

Es sind verschiedene Operationstechniken zur Implantation eines Fusionsimplantats bekannt. Der Zugang zur Bandscheibe kann von ventral erfolgen, um so die Gefahr einer Beschädigung des Rückenmarks zu vermeiden. Allerdings wird dazu ein langer Zugangsweg durch den Bauch- oder Brustraum des Patienten benötigt. Da es hierbei zu Komplikationen kommen kann, ist ein alternativer Zugangsweg von dorsal etabliert worden. Diese Operationstechniken von unmittelbar dorsal bzw. mehr von der Seite sind als PLIF (posterior lumbar intervertebral fusion) bzw. TLIF (transforaminelle interkorporelle lumbare Fusion) bekannt, bei der die Bandscheibe von posterior bzw. lateral exponiert wird. Aufgrund der kleinen Querschnitte beim Einsatz minimal invasiver Chirurgie ist die Größe der Fusionsimplantate stark beschränkt.

Die Implantation von Zwischenwirbelimplantaten gemäß einer PLIF erfolgt in einer sehr empfindlichen Region nahe des Rückenmarks, an der Nervenenden aus dem Spinalkanal der Wirbelsäule austreten. Eine Operation birgt daher regelmäßig das Risiko einer Verletzung dieser Nervenenden. Aus diesem Grund werden möglichst kleine Öffnungen durch Nutzung minimal invasiver Chirurgie zur Implantation geschaffen, was zur Folge hat, dass die Zwischenwirbelimplantate einerseits möglichst klein ausgeführt werden müssen. Andererseits sollen die Zwischenwirbelimplantate als Ersatz der Bandscheibe entweder eine möglichst große Oberfläche überdecken oder die Wirbel voneinander trennen bzw. einen festen Winkel zwischen den Wirbeln herstellen und die Wirbel abstützen.

Zwischenwirbelimplantate weisen daher Expansionseinrichtungen auf. Die Expansionseinrichtungen können beispielsweise den Abstand zwischen den Anlageflächen des Implantats an benachbarte Wirbel vergrößern. Alternativ können sie die Anlageflächen eines Implantats an die Wirbel vergrößern.

Es ist bekannt, für die Expansion der Zwischenwirbelimplantate Rastmechanismen vorzusehen. Rastmechanismen erlauben eine stufenweise Expansion des Zwischenwirbelimplantats. Eine kontinuierliche Verstellung des Zwischenwirbelimplantats für eine flexible und genaue Höhenverstellung ist mit Rastmechanismen nicht möglich.

Eine kontinuierliche Verstellung kann mittels Schneckenantrieben in Kombination mit Bewegungsgewinden oder der Zugkeiltechnik durchgeführt werden. Diese Vorrichtungen haben den Nachteil, dass sie lediglich einzelne Erfordernisse an die Mechaniken der Implantate erfüllen. Zu diesen Erfordernissen gehören eine hohe Belastbarkeit, eine hohe Kraftentwicklung bei der Expansion des Implantats und die Möglichkeit der Einstellung eines Lordosewinkels. Wenn der Lordosewinkel eingestellt werden kann, dann mangelt es an der Gesamthöhenverstellung des Implantats. Mechaniken mit hoher Belastbarkeit bei der Stützung der Wirbel weisen große Abmessungen auf, die einen Einsatz bei einer PLIF verhindern.

Aufgabe der Erfindung ist es damit, eine Vorrichtung bereitzustellen, die die oben genannten Nachteile vermeidet.

Die Aufgabe wird gelöst durch den Gegenstand der unabhängigen Ansprüche. Vorteilhafte Weiterbildungen sind Gegenstand der abhängigen Ansprüche.

Bei einem Zwischenwirbelimplantat umfassend mindestens zwei gegenüberliegende voneinander weg weisende Anlageflächen zur Anlage an zueinander weisende Wirbelkörperflächen zweier benachbarter Wirbel, wobei ein Abstand der beiden Anlageflächen mittels einer Expansionseinrichtung veränderlich ist, die zwischen den Anlageflächen angeordnet ist und mindestens ein Betätigungselement aufweist, ist erfindungsgemäß vorgesehen, dass die Expansionseinrichtung mindestens zwei über eine Drehachse scherenartig miteinander verbundene verschwenkbare Hebelelemente aufweist und die Expansionseinrichtung mindestens eine mittels des Betätigungselements betätigte Spreizkomponente, die zur Aufspreizung der Hebelelemente ausgebildet ist, und mindestens ein Sicherungselement umfasst, wobei das Sicherungselement zur Fixierung der minimalen Aufspreizung der Hebelelemente ausgebildet ist.

Zunächst seien einige Begriffe näher erläutert:
Unter einer Aufspreizung wird die Verschwenkung der Hebelelemente aus einem geschlossenen Zustand, der dem einer geschlossenen Schere entspricht, in einen aufgespreizten Zustand verstanden, der dem einer geöffneten Schere entspricht.

Unter einer minimalen Aufspreizung wird eine Aufspreizung der Hebelelemente verstanden, die nicht weiter verkleinert werden kann. D.h. die Hebelelemente können nicht weiter als bis zur minimalen Aufspreizung von dem aufgespreizten Zustand in den geschlossenen Zustand überführt werden.

Die Erfindung beruht auf dem Gedanken, mittels des Scherenmechanismus, der das Hebebühnenprinzip nutzt, eine Expansion des Zwischenwirbelimplantats zu bewirken. Dieses Prinzip wird durch die scherenartig über eine Drehachse miteinander verbundenen Hebelelemente umgesetzt. In dem geschlossenen Zustand weist das Zwischenwirbelimplantat eine geringe Höhe auf, so dass es durch eine kleine Öffnung mittels minimal invasiver Chirurgie zwischen die Wirbel eingeführt werden kann. In diesem Zustand ist der Scherenmechanismus geschlossen. Wenn das Zwischenwirbelimplantat an seine vorgesehene Position angeordnet ist, kann dessen Expansion durch die Aufspreizung der Hebelelemente in den aufgespreizten Zustand erfolgen.

Die Aufspreizung der Hebelelemente erfolgt mittels der Betätigung einer Spreizkomponente durch das Betätigungselement. Die Spreizkomponente übt auf Grund der Betätigung ein Drehmoment auf die Hebelelemente aus. Durch das Drehmoment werden die Hebelelemente um die Drehachse verschwenkt, und spreizen sich scherenartig auf. Die Aufspreizung der Hebelelemente bewirkt, dass der Abstand zwischen den Anlageflächen größer wird. Das Sicherungselement fixiert am Ende der Verschwenkung die minimale Aufspreizung der Hebelelemente. Solange das Betätigungselement nicht betätigt wird, kann die Aufspreizung der Hebelelemente höchstens bis zur durch das Sicherungselement festgelegten minimalen Aufspreizung verkleinert werden.

Die Spreizkomponente ist in der Lage, ein großes Drehmoment auf die Hebelelemente auszuüben. Damit kann genügend Kraft aufgewendet werden, um zwei Wirbel zu beabstanden. Weiter können die Auflageflächen und die Hebelelemente mit großen Kräften belastet werden. Der Scherenmechanismus des Expansionselements kann damit der Belastung durch die Wirbelsäule standhalten.

Die Erfindung ermöglicht weiter die Anpassung eines Lordosewinkels zwischen benachbarten Wirbeln mittels der Anpassung der Länge der Hebelarme der Hebelelemente. Unter einem Hebelarm wird das Teilstück des Hebelelements verstanden, das zwischen der Drehachse und einem Endabschnitt des Hebelelementes angeordnet ist. Im geschlossenen Zustand des Implantats sind aneinander liegende Hebelarme der beiden Hebelelemente gleich lang. Das Verhältnis der Längen der Hebelarme eines einzigen Hebelelements kann jedoch variieren, so dass bei einer Aufspreizung der Hebelelemente die einander gegenüberliegenden Anlageflächen des Zwischenwirbelimplantats gegeneinander verschwenkt werden.

Das erfindungsgemäße Zwischenwirbelimplantat kann zur Einstellung der Höhe zwischen benachbarten Wirbeln genutzt werden. Alternativ ist das Zwischenwirbelimplantat ausgebildet, die von ihm überspannte Auflagefläche zwischen benachbarten Wirbeln durch Expansion zu vergrößern. Die jeweilige Alternative wird durch die Ausrichtung des Implantats beim Einführen in den Zwischenwirbelraum festgelegt.

Beide Alternativen bewirken eine Fusion der benachbarten Wirbel. Diese Wirbel können sich nach der Implantation nicht mehr gegeneinander bewegen und bilden bei einer Bewegung der Wirbelsäule eine Einheit.

In einer ersten vorteilhaften Ausführungsform teilt die Drehachse die Hebelelemente in zwei Hebelarme gleicher Länge. Dies ermöglicht, die Wirbel mit parallel ausgerichteten Wirbelkörperoberflächen anzuheben.

In einer alternativen zweiten vorteilhaften Ausführungsform teilt die Drehachse die Hebelelemente in zwei Hebelarme unterschiedlicher Länge. Diese Ausführungsform ermöglicht eine konstruktive Einstellung eines Lordosewinkels zwischen den Wirbeln.

Mit Vorteil sind das Sicherungselement und das Betätigungselement einstückig ausgebildet. Zweckmäßigerweise ist das Sicherungselement dabei als eine Schraube und das Betätigungselement als der Kopf der Schraube ausgebildet. Die Spreizkomponente ist als mindestens zwei mit der Schraube als Spindeltrieb verbundene Keile ausgebildet. Die Schraube bildet in Kombination mit den Gewinden der Keile den Spindeltrieb. Spindeltriebe sind selbsthemmend ausgebildet, so dass eine Verstellung der Keile nur durch Betätigung des Kopfes der Schraube durchgeführt werden kann. Die Sicherung der Spreizkomponenten erfolgt in dieser Ausführungsform damit automatisch. Die Keile sind auf gegenüberliegenden Seiten der Drehachse zwischen den Hebelelementen angeordnet. Die Keile weisen jeweils eine Keilspitze auf, die in entgegengesetzte Richtungen weisen. Mit dieser Anordnung werden die Spreizelemente relativ zueinander entlang der Schraube bewegt, wenn die Schraube über das Betätigungselement gedreht wird. Die Keile werden mit ihren Keilspitzen zwischen die Hebelelemente gedrückt. Dies bewirkt eine Spreizung der scherenartig verbundenen Hebelelemente.

Vorteilhafterweise ist mindestens eine Anlagefläche an einem Brückenelement angeordnet, das mittels eines Festlagers an einem Hebelelement angeordnet ist, wobei das Brückenelement an dem anderen Hebelelement mittels eines Loslagers angeordnet ist. Im Gegensatz zu einer Anlagefläche, die an den Hebelelementen selbst angeordnet sein kann, erhält die an dem Brückenelement angeordnete Anlagefläche bei der Spreizung der Hebelelemente ihre Größe. Damit wird eine gleichmäßige Verteilung des Druckes auf die Wirbelkörperflächen ermöglicht. Weiter wird eine maximale Auflagefläche bereitgestellt, da das Brückenelement den sich durch die Aufspreizung der Hebelelemente ergebenden Freiraum zwischen den Hebelelementen überbrückt.

Mit Vorteil ist das Loslager als eine an einem Hebelelement angeordnete Gleitbahn ausgebildet. Das lose Ende des Brückenelements gleitet bei der Aufspreizung der Hebelelemente an der Gleitbahn entlang. Damit wird die Reibung zwischen dem Hebelelement und dem Brückenelement verringert, so dass die Aufspreizung einfacher erfolgen kann und Beschädigungen an dem Hebelelement oder dem Brückenelement vermieden werden.

Vorteilhafterweise liegen die Brückenelemente im geschlossenen Zustand bündig an dem Zwischenwirbelimplantat an. Weiter bilden die Hebelelemente und die Brückenelemente im geschlossenen Zustand mindestens eine geschlossene Lateralseite des Zwischenwirbelimplantats. Die Oberfläche des Zwischenwirbelimplantats weist keine Öffnungen auf. Damit weist das Zwischenwirbelimplantat eine "Stromlinien-Form" auf, die das Einführen des Zwischenwirbelimplantats in den Zwischenwirbelraum vereinfacht.

Weiter ist es zweckmäßig, dass die Lateralseite des Zwischenwirbelimplantats im aufgespreizten Zustand Durchbrechungen aufweist. In diese Durchbrechungen können Füllstoffe eingebracht werden, die das Knochenwachstum durch das Zwischenwirbelimplantat fördern.

Es ist zweckmäßig, dass die Anlageflächen eine strukturierte Oberfläche aufweisen. Die Anlageflächen haben durch die strukturierten Oberflächen einen erhöhten Halt an den Wirbelkörperflächen. Ein Ab- oder Wegrutschen des Implantats wird damit vermieden.

Zweckmäßigerweise ist weiter ein Teil der Anlagefläche an den Endbereichen der Hebelelemente angeordnet und im geschlossenen Zustand um eine Mittelachse des Zwischenwirbelimplantats geneigt. Der Winkel der Neigung des Teils der Anlagefläche beträgt vorteilhafterweise zwischen 5° bis 30°, vorzugsweise zwischen 10° bis 20°, weiter vorzugsweise 15°. Damit wird im aufgespreizten Zustand eine parallele Ausrichtung der Anlagefläche zu den Wirbelkörperoberflächen bewirkt. Die Wirbelkörperfläche liegt damit sicher an den Anlageflächen an.

Weiter umfasst das Zwischenwirbelimplantat vorteilhafterweise eine Schlagfläche, die zur Aufnahme von Hammerschlägen ausgebildet ist. Damit kann das Implantat mittels eines Hammers in den Wirbelzwischenraum eingeführt werden.

Mit Vorteil besteht das Zwischenwirbelimplantat aus Titan, einer Titanlegierung, CoCr oder PEEK. Diese Materialien vermeiden Komplikationen und Abstoßungsreaktionen bei einem Patienten.

Die Erfindung betrifft weiter einen Satz von Zwischenwirbelimplantaten, die gemäß Anspruch 1 ausgebildet sind, wobei sich die Zwischenwirbelimplantate in dem Längenverhältnis der zwei Hebelarme eines Hebelelements unterscheiden.

Mindestens ein Zwischenwirbelimplantat kann gemäß mindestens einem der Merkmale der oben angeführten Beschreibung ausgebildet sein.

Mittels der beigefügten Zeichnung, die vorteilhafte Ausführungsformen darstellt, wird die Erfindung näher erläutert. Es zeigen:
- Fig. 1a, b, c:: eine schematische Darstellung eines Zwischenwirbelimplantats im geschlossenen Zustand (a), aufgespreizten Zustand (b) und mit einer Lordosewinkel-Einstellung (c);
- Fig. 2a, b, c:: eine schematische Darstellung von Komponenten des Zwischenwirbelimplantats; und
- Fig. 3a, b:: eine schematische Darstellung eines im Zwischenwirbelraum eingebrachten Zwischenwirbelimplantats im geschlossenen (a) und gespreizten (b) Zustand.

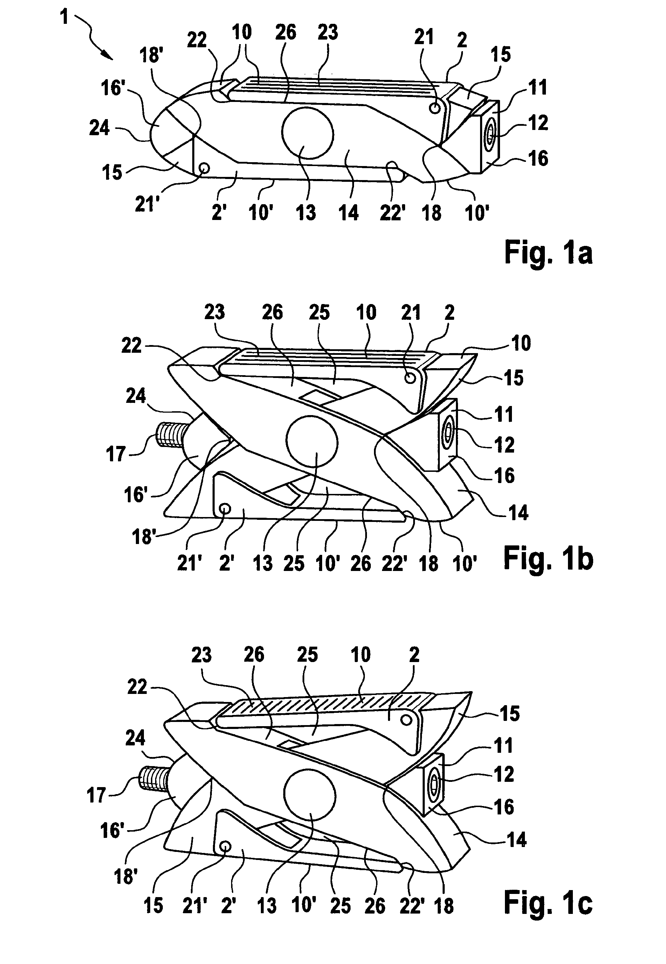

Das Zwischenwirbelimplantat wird in seiner Gesamtheit mit dem Bezugszeichen 1 bezeichnet. Es umfasst zwei Hebelelemente 14, 15, die mittels einer Drehachse 13 scherenartig miteinander verbunden sind. In Figur 1a wird das Zwischenwirbelimplantat 1 im geschlossenen Zustand dargestellt. Die Drehachse 13 teilt beide Hebelelemente 14, 15 in gleich große Hälften. Jede der Hälften ist ein Hebelarm. In den hier beschriebenen Ausführungsformen sind im geschlossenen Zustand nebeneinander angeordnete Hebelarme verschiedener Hebelelemente 14, 15 gleich lang. Sie können jedoch auch verschiedene Längen aufweisen.

Zwischen im geschlossenen Zustand nebeneinander angeordneten Hebelarmen der Hebelelemente 14, 15 sind Spreizkomponenten 16, 16' an einander gegenüberliegenden Seiten des Zwischenwirbelimplantats 1 angeordnet. Die Spreizkomponenten 16, 16' können mittels eines Betätigungselements 12 aufeinander zu bewegt werden. Dabei werden sie auf die Drehachse 13 zu bewegt und weiter zwischen die Hebelarme der Hebelelemente 14, 15 gedrückt. Das Betätigungselement 12 ist dabei an einem der Spreizkomponenten 16, 16' angeordnet.

Die Spreizkomponenten 16, 16' sind keilförmig ausgebildet. Die Keilspitzen 18, 18' sind auf die jeweils andere Spreizkomponente 16, 16' ausgerichtet und weisen weiter zwischen die Hebelelemente 14, 15. Die an den Keilspitzen 18, 18' anliegenden Seitenwände der Spreizkomponenten 16, 16' liegen an den Hebelelementen 14, 15 an. Mittels der Seitenwände üben die Spreizkomponenten 16, 16' während des Eindringens zwischen die Hebelelemente 14, 15 ein Drehmoment auf die Hebelelemente 14, 15 aus. Das Drehmoment bewirkt eine Aufspreizung der Hebelelemente 14, 15. In Figur 1b ist der aufgespreizte Zustand des in Figur 1a gezeigten Zwischenwirbelimplantats 1 dargestellt.

An den Endabschnitten der Hebelarme 14, 15 sind Anlageflächen 10, 10' an Wirbelkörperflächen 30, 30' von benachbarten Wirbeln 3, 3' angeordnet. Im aufgespreizten Zustand gemäß Figur 1b sind die Anlageflächen 10, 10' im Vergleich zum geschlossenen Zustand gemäß Figur 1a weiter voneinander beabstandet.

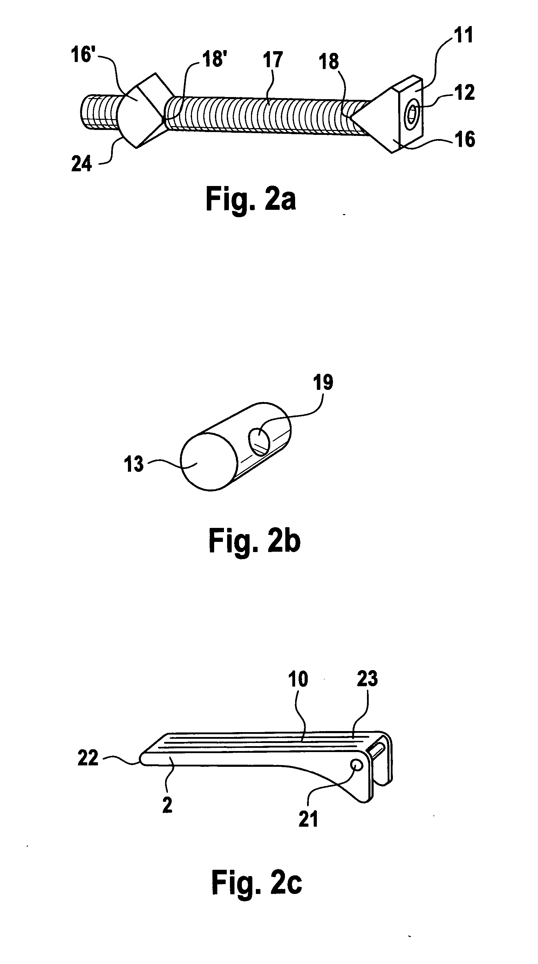

Die Spreizkomponenten 16, 16' weisen Innengewinde auf. Wie in Figur 2a dargestellt, sind sie über eine Schraube miteinander verbunden, die als Sicherungselement 17 fungiert. Die Innengewinde und die Schraube bilden einen Spindeltrieb. Die Schraube weist einen Kopf auf, der als Betätigungselement 12 fungiert. Eine Betätigung des Betätigungselements 12 bewirkt eine Drehung der Schraube, wodurch die Spreizkomponenten 16, 16' aufeinander zu bewegt oder voneinander weg bewegt werden können. Ohne Drehung der Schraube als Sicherungselement 17 ändert sich der Abstand zwischen den Spreizkomponenten 16, 16' auf Grund der Selbsthemmung des Spindeltriebs, der die Position der Spreizkomponenten 16, 16' festlegt, nicht. Damit wird die minimale Aufspreizung der Hebelelemente 14, 15 durch das Sicherungselement 17 festgelegt. Die Spreizkomponenten 16, 16' verbleiben an ihrer Position zwischen den Hebelelementen 14, 15. In Kombination mit dem Druck der Wirbel 3, 3' auf die Anlageflächen 10, 10', der ein Drehmoment bewirkt, der das Zwischenwirbelimplantat 1 von dem aufgespreizten Zustand in den geschlossenen Zustand überführt, wird eine weitere Verschwenkung der Hebelelemente 14, 15 verhindert.

Die Drehachse 13 der Hebelelemente 14, 15 weist gemäß Figur 2b eine Führungsbohrung 19 auf. Das Sicherungselement 17 ist durch diese Bohrung 19 durchgeführt. Die Spreizkomponenten 16, 16' sind auf den gegenüberliegenden Seiten der Drehachse 13 an dem Sicherungselement 17 angeordnet. Mit der Führungsbohrung 19 werden die Spreizkomponenten 16, 16', das Sicherungselement 17 und das Betätigungselement 12 zwischen die Hebelelemente 14, 15 geführt.

In Figur 1c ist eine Ausführungsform eines Zwischenwirbelimplantats 1 dargestellt, dessen Hebelelemente 14, 15 durch die Drehachse 13 in verschieden große Hebelarme geteilt werden. Bei einer Aufspreizung der Hebelelemente 14, 15 werden die Endabschnitte der längeren Hebelarme weiter voneinander beabstandet als die Endabschnitte der kurzen Hebelarme. Dadurch entsteht ein Winkel zwischen den Auflageflächen 10, 10'. Mit dieser Ausführungsform des Zwischenwirbelimplantats 1 kann ein Lordosewinkel zwischen benachbarten Wirbeln 3, 3' eingestellt werden.

Das Zwischenwirbelimplantat 1 weist weiter Brückenelemente 2, 2' auf. Ein Beispiel eines Brückenelements 2, 2' ist in Figur 2c dargestellt. Die Brückenelemente 2, 2' umfassen einen Teil der Anlageflächen 10, 10'. Sie sind über Festlager 21, 21' mit einem der Hebelelemente 14, 15 drehbar verbunden. Auf dem jeweils anderen Hebelelement 14, 15 sind sie über ein Loslager 22, 22' gelagert. Das Loslager 22, 22' wird durch eine Gleitbahn 26 gebildet, die an dem jeweils anderen Hebelelement 14, 15 angeordnet ist. Bei der Aufspreizung des Zwischenwirbelimplantats 1 gleiten die Brückenelemente 2, 2' über das als Gleitbahn 26 ausgebildete Loslager 22, 22' an dem jeweiligen Hebelelement 14, 15 entlang. Dabei werden die Brückenelemente 2, 2' um die Festlager 21, 21' gedreht. Die Brückenelemente 2, 2' überbrücken damit die Strecke zwischen den Hebelelementen 14, 15. Dadurch entstehen Durchbrechungen 25 in der Lateralseite des Zwischenwirbelimplantats 1. In diese Durchbrechungen können Stoffe eingebracht werden, die das Knochenwachstum fördern.

Die Größe der an den Brückenelementen 2, 2' angeordneten Anlageflächen 10, 10' wird durch den Expansionsvorgang im Gegensatz zu den an den Hebelelementen 2, 2' angeordneten Anlageflächen 10, 10' nicht verändert. Damit wird den Wirbelkörperoberflächen 30, 30' der anliegenden Wirbel 3, 3' eine maximale Anlagefläche 10, 10' bereitgestellt.

Die Anlageflächen 10, 10' weisen strukturierte Oberflächen 23 auf. Mittels der strukturierten Oberflächen 23 wird der Kontakt zu den Wirbelkörperflächen 30, 30' der Wirbel 3, 3' verbessert. Weiter vermeidet die strukturierte Oberfläche 23 ein Abrutschen des Zwischenwirbelimplantats 1 an der jeweiligen Knochenoberfläche. Die Struktur einer strukturierten Oberfläche 23 kann Querrillen, Längsrillen oder Spikes umfassen.

Weiter ist der Teil der Anlageflächen 10, 10', der an den Enden der Hebelelemente 14, 15 angeordnet ist, im geschlossenen Zustand des Zwischenwirbelimplantats 1 um 15° gegen eine Mittelachse des Zwischenwirbelimplantats 1 geneigt ausgebildet. Bei der Überführung des Zwischenwirbelimplantats 1 in den aufgespreizten Zustand werden die Anlageflächen 10, 10' in eine zu den Wirbelkörperoberflächen 30, 30' parallele Ausrichtung geneigt, so dass die Anlagefläche 10, 10' maximal ist.

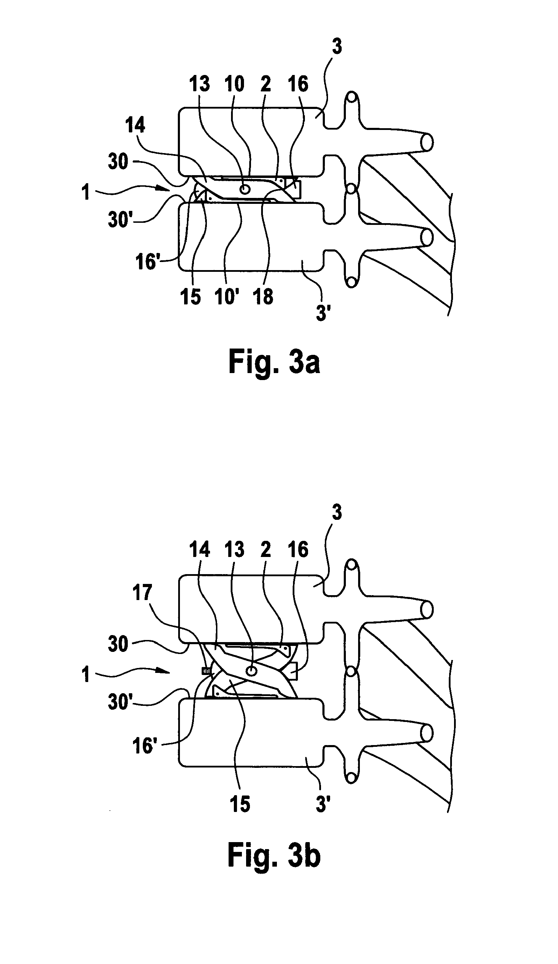

Die Figuren 3a und 3b zeigen die Aufspreizung eines Zwischenwirbelimplantats 1 mit einem an dem oberen Wirbel 3 angeordneten Brückenelement 2. Dazu muss das Zwischenwirbelimplantat 1 zunächst im geschlossenen Zustand zwischen die Wirbel 3, 3' angeordnet werden. Wenn es an der vorgesehenen Position angelangt ist, wird die Expansionseinrichtung des Zwischenwirbelimplantats 1 mithilfe des Betätigungselements 12 betätigt, so dass das Zwischenwirbelimplantat 1 expandiert wird.

Das Zwischenwirbelimplantat 1 kann ebenso um 90° um das Sicherungselement 17 verdreht eingesetzt werden. Die Betätigung des Betätigungselements 12 resultiert dann in einer Aufspreizung des Zwischenwirbelimplantats 1 entlang der Wirbelkörperflächen 30, 30'.

Das Zwischenwirbelimplantat 1 weist weiter eine Schlagfläche 11 auf, die ausgebildet ist, Hammerschläge aufzunehmen. Die Schlagfläche 11 ist an der Spreizkomponente 16, 16' angeordnet, an der das Betätigungselement 12 angeordnet ist. Das Zwischenwirbelimplantat 1 kann damit mittels eines Hammers in einen Wirbelzwischenraum eingeführt werden. Weiter bleibt das Betätigungselement 12 durch die Anordnung an der Spreizkomponente 16, 16', an der die Schlagfläche 11 angeordnet ist, von außen erreichbar und kann weiterhin betätigt werden.

Die Spreizkomponente 16, 16', die keine Schlagfläche 11 aufweist, umfasst eine abgerundete Rückwand 24. Die Rückwand 24 liegt der Keilspitze 18, 18' der Spreizkomponente 16, 16' gegenüber. Durch die Abrundung der Rückwand 24 wird die Einführung des Zwischenwirbelimplantats 1 in den Raum zwischen den Wirbeln 3, 3' erleichtert.

Das Zwischenwirbelimplantat 1 weist mit Vorteil eine Gesamtlänge zwischen 15 mm und 30 mm, vorzugsweise 23 mm, auf. Weiter weist das Zwischenwirbelimplantat 1 mit Vorteil eine Höhe zwischen 7 mm und 12 mm, vorzugsweise 7 mm, auf. Es ist weiter zweckmäßig, dass das Zwischenwirbelimplantat eine Breite zwischen 7 mm und 20 mm, vorzugsweise 7 mm, aufweist. Zwischenwirbelimplantate mit diesen Maßen benötigen bei der Implantation nur kleine Schnitte, um zwischen die Wirbel 3, 3' eingefügt zu werden.

Die Expansionseinrichtung ist weiter so ausgebildet, dass die Anlageflächen 16, 16' bis zu 14 mm voneinander beabstandbar sind. Weiter besteht das Zwischenwirbelimplantat aus Titan, CoCr oder PEEK.

## Patentansprüche

1. Zwischenwirbelimplantat umfassend mindestens zwei gegenüberliegende voneinander weg weisende Anlageflächen (10, 10') zur Anlage an zueinander weisende Wirbelkörperflächen (30, 30') zweier benachbarter Wirbel (3, 3'), wobei ein Abstand der beiden Anlageflächen (10, 10') mittels einer Expansionseinrichtung veränderlich ist, die zwischen den Anlageflächen (10, 10') angeordnet ist und mindestens ein Betätigungselement (12) aufweist,
**dadurch gekennzeichnet, dass**
die Expansionseinrichtung mindestens zwei über eine Drehachse (13) scherenartig miteinander verbundene verschwenkbare Hebelelemente (14, 15) aufweist und die Expansionseinrichtung mindestens eine mittels des Betätigungselements betätigte Spreizkomponente (16, 16'), die zur Aufspreizung der Hebelelemente (14, 15) ausgebildet ist, und mindestens ein Sicherungselement (17) umfasst, wobei das Sicherungselement (17) zur Fixierung der minimalen Aufspreizung der Hebelelemente (14, 15) ausgebildet ist.

2. Zwischenwirbelimplantat nach Anspruch 1,
**dadurch gekennzeichnet, dass**
die Drehachse (13) die Hebelelemente (14, 15) in zwei Hälften gleicher Länge teilt.

3. Zwischenwirbelimplantat nach Anspruch 1,
**dadurch gekennzeichnet, dass**
die Drehachse (13) die Hebelelemente (14, 15) in zwei Hälften unterschiedlicher Länge teilt.

4. Zwischenwirbelimplantat nach einem der Ansprüche 1 bis 3,
**dadurch gekennzeichnet, dass**
das Sicherungselement (17) und das Betätigungselement 12) einstückig ausgebildet sind.

5. Zwischenwirbelimplantat nach einem der Ansprüche 1 bis 4,
**dadurch gekennzeichnet, dass**
das Sicherungselement (17) als eine Schraube und das Betätigungselement (12) als ein Kopf der Schraube ausgebildet ist, und die Spreizkomponente (16, 16') als mindestens zwei mit der Schraube als Spindeltrieb verbundene Keile ausgebildet ist, wobei die Keile auf den gegenüberliegenden Seiten der Drehachse (13) zwischen den Hebelelementen angeordnet sind und die Keile jeweils eine Keilspitze (18, 18') aufweisen, die in entgegengesetzte Richtungen weisen.

6. Zwischenwirbelimplantat nach einem der Ansprüche 1 bis 5,
**dadurch gekennzeichnet, dass**
mindestens eine Anlagefläche (10, 10') an einem Brückenelement (2, 2') angeordnet ist, das mittels eines Festlagers (21, 21') an einem der Hebelelemente (14, 15) angeordnet ist, wobei das Brückenelement (2, 2') an dem jeweils anderen Hebelelement (14, 15) mittels eines Loslagers (22, 22') angeordnet ist.

7. Zwischenwirbelimplantat nach Anspruch 6,
**dadurch gekennzeichnet, dass**
das Loslager (22, 22') an dem jeweils anderen Hebelelement (14, 15) als Gleitbahn (26) ausgebildet ist.

8. Zwischenwirbelimplantat nach einem der Ansprüche 6 bis 7,
**dadurch gekennzeichnet, dass**
die Brückenelemente (2, 2') im geschlossenen Zustand bündig an dem Zwischenwirbelimplantat (1) anliegen.

9. Zwischenwirbelimplantat nach einem der Ansprüche 6 bis 8,
**dadurch gekennzeichnet, dass**
die Hebelelemente (14, 15) und die Brückenelemente (2, 2') im geschlossenen Zustand mindestens eine geschlossene Lateralseite des Zwischenwirbelimplantats (1) bilden.

10. Zwischenwirbelimplantat nach Anspruch 9,
**dadurch gekennzeichnet, dass**
die Lateralseite des Zwischenwirbelimplantats (1) im aufgespreizten Zustand Durchbrechungen (25) aufweist.

11. Zwischenwirbelimplantat nach einem der Ansprüche 1 bis 10,
**dadurch gekennzeichnet, dass**
das Zwischenwirbelimplantat (1) eine Gesamtlänge zwischen 15 und 30 mm, vorzugsweise 23 mm, aufweist.

12. Zwischenwirbelimplantat nach einem der Ansprüche 1 bis 11,
**dadurch gekennzeichnet, dass**
das Zwischenwirbelimplantat (1) eine Höhe zwischen 7 und 12 mm, vorzugsweise 7 mm, aufweist.

13. Zwischenwirbelimplantat nach einem der Ansprüche 1 bis 12,
**dadurch gekennzeichnet, dass**
das Zwischenwirbelimplantat (1) eine Breite zwischen 7 und 20 mm, vorzugsweise 7 mm, aufweist.

14. Zwischenwirbelimplantat nach einem der Ansprüche 1 bis 13,
**dadurch gekennzeichnet, dass**
die Anlageflächen (10, 10') bis zu 14 mm voneinander beabstandbar sind.

15. Zwischenwirbelimplantat nach einem der Ansprüche 1 bis 14,
**dadurch gekennzeichnet, dass**
die Anlageflächen (10, 10') eine strukturierte Oberfläche (23) aufweisen.

16. Zwischenwirbelimplantat nach einem der Ansprüche 1 bis 15,
**dadurch gekennzeichnet, dass**
ein Teil der Anlagefläche (10, 10') an den Endbereichen der Hebelelemente (14, 15) angeordnet ist und im geschlossenen Zustand um eine Mittelachse des Zwischenwirbelimplantats (1) geneigt ist.

17. Zwischenwirbelimplantat nach Anspruch 16,
**dadurch gekennzeichnet, dass**
der Winkel der Neigung des Teils der Anlagefläche (10, 10') zwischen 5° bis 30°, vorzugsweise zwischen 10° bis 20°, weiter vorzugsweise 15°, beträgt.

18. Zwischenwirbelimplantat nach einem der Ansprüche 1 bis 17,
**dadurch gekennzeichnet, dass**
das Zwischenwirbelimplantat (1) eine Schlagfläche (11) umfasst, die als Auftrefffläche für Hammerschläge ausgebildet ist.

19. Zwischenwirbelimplantat nach einem der Ansprüche 1 bis 18,
**dadurch gekennzeichnet, dass**
das Zwischenwirbelimplantat (1) aus Titan, einer Titanlegierung, CoCr oder PEEK besteht.

20. Satz von Zwischenwirbelimplantaten nach Anspruch 1,
**dadurch gekennzeichnet, dass**
die Zwischenwirbelimplantate (1) sich in dem Längenverhältnis der zwei Hebelarme eines Hebelelements (14, 15) unterscheiden.

21. Satz von Zwischenwirbelimplantaten nach Anspruch 20,
**dadurch gekennzeichnet, dass**
mindestens ein Zwischenwirbelimplantat (1) nach einem der Ansprüche 2 bis 19 ausgebildet ist.
